# EUROPEAN PATENT SPECIFICATION

(11) **EP 4 085 956 B1**
(45) Date of publication and mention of the grant of the patent: **07.08.2024**
(21) Application number: 19958510.0
(22) Date of filing: 30.12.2019
(51) Int. Cl.: A61M 16/01, A61M 16/00, A61M 16/10, A61M 16/20

(54) **BACKUP FLOW CONTROL SYSTEM AND METHOD APPLICABLE TO ANESTHESIA MACHINE**
BACKUP-FLUSSSTEUERUNGSSYSTEM UND -VERFAHREN FÜR ANÄSTHESIEGERÄT
SYSTÈME DE SECOURS DE COMMANDE D'ÉCOULEMENT ET PROCÉDÉ APPLICABLE À UNE MACHINE D'ANESTHÉSIE

(43) Date of publication of application: 09.11.2022
(73) Proprietor: Shenzhen Mindray Bio-Medical Electronics Co., Ltd., Shenzhen, Guangdong 518057 (CN)
(72) Inventor: LIANG, Dongsheng, Shenzhen, Guangdong 518057 (CN); HU, Min, Shenzhen, Guangdong 518057 (CN)
(74) Representative: KIPA AB
(86) International application number: PCT/CN2019/130185
(87) International publication number: WO 2021/134352

(56) References cited:
- WO-A1-2009/006932
- WO-A2-2014/178730
- CN-A- 103 800 975
- CN-A- 103 800 983
- CN-A- 103 800 984
- CN-A- 103 809 620
- CN-A- 103 809 621
- CN-A- 103 809 622
- CN-A- 109 771 767
- CN-U- 202 305 529
- US-A1- 2014 130 906
- US-A1- 2015 136 235

## Description

### TECHNICAL FIELD

Embodiments of the disclosure relate to the technical field of medical devices, and more particularly, to a backup flow control system and method applicable to an anesthesia machine.

### BACKGROUND

Two ways to control a fresh gas flow of an anesthesia machine are provided, one is to use a mechanical needle valve to regulate the flow, and the other is to use an electronic flow control system to control the flow, namely, the system automatically controls a flow of a gas circuit valve so as to reach an artificially set target flow or oxygen concentration. The electronic flow control system, due to its high accuracy of flow control, is widely applied in various types of anesthesia machines.

In case of power failure and electronic device failure in the above-mentioned electronic flow control system, the anesthesia machine may not provide oxygen for a patient. Therefore, in general, it is possible to configure a backup flow control system for the electronic flow control system of the anesthesia machine, so as to achieve emergency ventilation in case of the failure of the electronic flow control system and to improve safety.

At present, mainly three types of backup flow control systems are provided. In a first type of backup flow control system, a needle valve for emergency regulation is directly exposed outside the anesthesia machine, which is prone to accidental regulation and influencing a patient's ventilation. In a second type of backup flow control system, a needle valve is hidden under a cover, and when emergency ventilation is required, the cover is opened for switching to emergency ventilation, and a flow is then regulated by using the needle valve; however, it may be difficult for medical care personnel to find the cover in an emergency state, resulting in a delay in treatment. In a third type of backup flow control system, a needle valve is retracted inside an anesthesia machine, and when the electronic flow control system fails or is manually operated, the needle valve pops up for regulation, electronic control and manual control of popup and retraction of the needle valve need to be compatible, which resulting in a complicated structure. Therefore, the existing three types of backup flow control systems have low flexibility and reliability. Patent applications US 2014/130906 A1 and WO 2009/006932 A1 provide teachings related to the technical field of the application.

### SUMMARY

In order to solve the above-mentioned technical problems, embodiments of the disclosure are expected to provide a backup flow control system and method applicable to an anesthesia machine, which not only protects a flow regulation valve from accidental regulation by means of an openable cover, but also is simple in structure, and is capable of automatically opening the cover when backup ventilation is required, thereby improving flexibility and reliability.

The technical solutions of the embodiments of the disclosure are implemented as follows.

An embodiment of the disclosure provides a backup flow control system applicable to an anesthesia machine as set forth in claim 1.

The above-mentioned system further includes an electromagnet and a permanent magnet, where power sources for the electromagnet and the electronic flow control system are different, one of the permanent magnet and the electromagnet is fixed to the cover and the other one thereof is fixed to the accommodation cavity, and the permanent magnet corresponds to the electromagnet in position; and the first controller is connected to the electromagnet and controls to energize and de-energize of the electromagnet.

The first controller controls to energize the electromagnet when the electronic flow control system is in the failure state, such that the magnetism generated by a magnetic pole at an end of the electromagnet close to the permanent magnet is the same as the magnetism at an end of the permanent magnet close to the electromagnet in order to separate the electromagnet from the permanent magnet, and controls to de-energize the electromagnet when the electronic flow control system is in the working state.

In the above-mentioned system, the failure state is at least one of: communication failure, valve failure, sensor failure, anesthesia machine power system failure and user interface failure of the electronic flow control system.

The above-mentioned system further includes a position sensor,
where the position sensor is connected to the first controller and fixed to the cover or the accommodation cavity; and
the first controller controls to turn on the backup ventilation mode when the position sensor detects that the cover is opened.

In the above-mentioned system, the position sensor is at least one of: a hall sensor, a photoelectric coupling sensor and a position switch.

In the above-mentioned system, the first controller controls to turn on the backup ventilation mode when it is detected that the electromagnet generates a separating induction current, and controls to turn off the backup ventilation mode when it is detected that the electromagnet generates an attracting induction current.

In the above-mentioned system, the flow regulation valve regulates a flow of a gas delivered to a patient according to a received regulation operation, when the backup ventilation mode is turned on, and stores a basic flow therein, when the backup ventilation mode is turned off.

In the above-mentioned system, the gas is oxygen, or oxygen and air.

The above-mentioned system further includes a system switch,
where the system switch is connected to the flow regulation valve; and
the system switch prevents leakage of the basic flow stored in the flow regulation valve, when in the closed state.

The above-mentioned system further includes a flow sensor,
where the flow sensor is connected to the flow regulation valve and the first controller, so as to detect a flow through the flow regulation valve and to feedback the detected flow to the first controller.

The above-mentioned system further includes a glass tube flow meter,
where the glass tube flow meter is connected to the flow regulation valve, so as to display a gas flow regulated by the flow regulation valve, when the backup ventilation mode is turn.

The above-mentioned system further includes a glass tube backlight,
where the glass tube backlight is disposed at a back of the glass tube flow meter and is connected to the first controller, and
the first controller controls the glass tube backlight to emit light when controlling to turn on the backup ventilation mode.

In the above-mentioned system, power sources for the electromagnet and the glass tube backlight are different from a power source for the electronic flow control system.

In the above-mentioned system, the first controller is also included in the electronic flow control system,
and the first controller detects an operating state of the electronic flow control system.

In the above-mentioned system, the electromagnet is energized and separated from the permanent magnet, when the first controller fails, thereby controlling to open the cover.

In the above-mentioned system, the electronic flow control system includes a second controller,
where the first controller is connected to the second controller; and
the second controller detects an operating state of the electronic flow control system and transmits the detected operating state to the first controller.

In the above-mentioned system, the first controller determines that the operating state of the electronic flow control system is a failure state when the interruption of communication with the second controller is detected.

An embodiment of the disclosure provides a backup flow control method applicable to an anesthesia machine as set forth in claim 10.

In the above-mentioned method, controlling, by the first controller, the backup flow control system to turn on the backup ventilation mode and to open the cover that closes the accommodation cavity, when the electronic flow control system is in the failure state, or controlling, by the first controller, the backup flow control system to turn off the backup ventilation mode when the electronic flow control system is in the working state includes:
controlling, by the first controller, to energize and de-energize an electromagnet according to the operating state of the electronic flow control system;
where one of a permanent magnet and the electromagnet is fixed to the cover, the other one thereof is fixed to the accommodation cavity, the permanent magnet corresponds to the electromagnet in position, and the first controller is connected to the electromagnet.

In the above-mentioned method, controlling, by the first controller, to energize and de-energize the electromagnet according to the operating state of the electronic flow control system includes:
controlling, by the first controller, to energize the electromagnet when the electronic flow control system is in the failure state, such that the magnetism generated by a magnetic pole at an end of the electromagnet close to the permanent magnet is the same as the magnetism generated by a magnetic pole at an end of the permanent magnet close to the electromagnet in order to separate the electromagnet from the permanent magnet, and controlling, by the first controller, to de-energize the electromagnet when the electronic flow control system is in the working state.

The above-mentioned method further includes:
controlling, by the first controller, to turn on the backup ventilation mode when a position sensor detects that the cover is opened.

The above-mentioned method further includes:
controlling, by the first controller, to turn on the backup ventilation mode when it is detected that the electromagnet generates a separating induction current, and controlling, by the first controller, to turn off the backup ventilation mode when it is detected that the electromagnet generates an attracting induction current.

In the above-mentioned method, if the first controller is also included in the electronic flow control system, obtaining the operating state of the electronic flow control system by the first controller includes:
detecting the operating state of the electronic flow control system by the first controller.

In the above-mentioned method, the electronic flow control system includes a second controller, the first controller being connected to the second controller, and obtaining the operating state of the electronic flow control system by the first controller includes:
detecting the operating state of the electronic flow control system by the second controller; and
receiving, by the first controller, the operating state detected by the second controller.

In the above-mentioned method, obtaining the operating state of the electronic flow control system by the first controller further includes:
determining, by the first controller, that the operating state of the electronic flow control system is a failure state when interruption of communication with the second controller is detected.

An embodiment of the disclosure provides a backup flow control system applicable to an anesthesia machine. The backup flow control system turns on a backup ventilation mode when an electronic flow control system of the anesthesia machine has failed. The backup flow control system includes: a first controller, a flow regulation valve, an accommodation cavity and a cover, where the accommodation cavity is a hollow structure having an opening, the cover is connected to the opening of the accommodation cavity to close the opening, and the flow regulation valve is disposed inside the accommodation cavity; and the first controller, according to an operating state of the electronic flow control system, controls to turn on the backup ventilation mode and to open the cover, such that the opening opens to expose the flow regulation valve, or controls to turn off the backup ventilation mode. The backup flow control system provided by the embodiment of the disclosure not only protects the flow regulation valve from accidental regulation by the openable cover, but also has a simple structure, and is capable of automatically opening the cover when backup ventilation is required, thereby improving flexibility and reliability.

### BRIEF DESCRIPTION OF THE DRAWINGS

FIG. 1 is a first schematic structural diagram of a backup flow control system applicable to an anesthesia machine according to an embodiment of the disclosure;
FIG. 2 is a second schematic structural diagram of a backup flow control system applicable to an anesthesia machine according to an embodiment of the disclosure;
FIG. 3 is a third schematic structural diagram of a backup flow control system applicable to an anesthesia machine according to an embodiment of the disclosure;
FIG. 4 is a first schematic diagram of a connection between an exemplary backup flow control system and an electronic flow control system according to an embodiment of the disclosure;
FIG. 5 is a second schematic diagram of a connection between an exemplary backup flow control system and an electronic flow control system according to an embodiment of the disclosure; and
FIG. 6 is a schematic flow diagram of a backup flow control method applicable to an anesthesia machine according to an embodiment of the disclosure.

### DESCRIPTION OF THE PREFERRED EMBODIMENTS

The description has been made with reference to various example embodiments herein. However, persons skilled in the art should appreciate that changes and modifications may be made to the example embodiments without departing from the scope herein. For example, various operation steps and assemblies for executing operation steps may be implemented in different ways according to a specific application or considering any number of cost functions associated with the operation of the system (for example, one or more steps may be deleted, modified, or incorporated into other steps).

The terms "first", "second" and the like in the specification and the claims of the disclosure and the above drawings are used to distinguish different objects, but not to describe a specific order. In addition, the terms "include", "have", and any variations thereof are intended to cover non-exclusive inclusion. For example, a process, a method, a system, a product, or a device that includes a series of steps or units is not limited to the listed steps or units, but optionally further includes unlisted steps or units, or optionally further includes other steps or units inherent in these processes, methods, or devices.

In order to understand the features and technical contents of embodiments of the disclosure in more detail, the implementation of the embodiments of the disclosure will be described below in detail with reference to the accompanying drawings, which are for reference and illustration only, and are not intended to limit the embodiments of the disclosure.

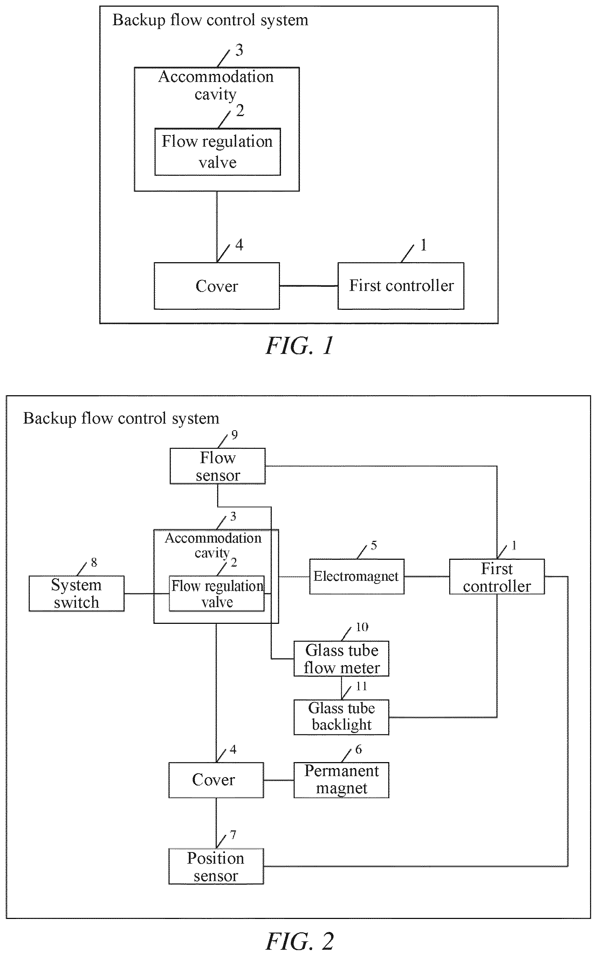

The embodiments of the disclosure provide a backup flow control system applicable to an anesthesia machine. The backup flow control system enters a backup ventilation mode when an electronic flow control system of the anesthesia machine has failed. FIG. 1 is a first schematic structural diagram of a backup flow control system applicable to an anesthesia machine according to an embodiment of the disclosure. As shown in FIG. 1, the backup flow control system includes: a first controller 1, a flow regulation valve 2, an accommodation cavity 3 and a cover 4.

Specifically, in this embodiment of the disclosure, the accommodation cavity 3 is a hollow structure having an opening, the cover 4 is connected to the opening of the accommodation cavity 3 to close the opening, and the flow regulation valve is disposed inside the accommodation cavity.

The first controller 1, according to an operating state of the electronic flow control system, controls to turn on a backup ventilation mode and to open the cover 4, such that the opening opens to expose the flow regulation valve 2; or controls to turn off the backup ventilation mode.

It should be noted that one or more first controllers 1 and one or more flow regulation valves 2 may be provided in this embodiment of the disclosure. The specific number of the first controllers 1 and the specific number of the flow regulation valves 2 may be set according to actual requirements, and are not limited in the embodiment of the disclosure.

It should be noted that in the embodiment of the disclosure, the flow regulation mode 2 may be specifically a mechanical needle valve configured to regulate a flow. Of course, the flow regulation valve 2 may also be other types of devices configured to achieve flow regulation, which are not limited in the embodiment of the disclosure.

It should be noted that one or more first controllers 1 may be provided in the embodiment of the disclosure. In a case where a plurality of first controllers 1 are provided, the plurality of first controllers 1 may monitor their operating states mutually, such that normal first controllers 1 may be used to turn on or off the backup ventilation mode in the event of failure of some of the first controllers 1. The specific number of the first controllers 1 may be set according to actual requirements, and is not limited in the embodiment of the disclosure.

It will be appreciated that in the embodiment of the disclosure, the first controller 1 may control opening and closing of the cover 4 according to the operating state of the electronic flow control system such that the cover may be automatically opened to facilitate an operation by a user when backup ventilation is required. The way in which the first controller 1 achieves control of the opening and closing of the cover is not limited in the embodiment of the disclosure.

FIG. 2 is a second schematic structural diagram of a backup flow control system applicable to an anesthesia machine according to an embodiment of the disclosure. As shown in FIG. 2, in this embodiment of the disclosure, the backup flow ventilation system further includes an electromagnet 5 and a permanent magnet 6, where power sources for the electromagnet 5 and the electronic flow control system are different, one of the permanent magnet 6 and the electromagnet 5 is fixed to the cover 4, and the other one thereof is fixed to the accommodation cavity 3, and the permanent magnet 6 corresponds to the electromagnet 5 in position. The first controller 1 is connected to the electromagnet 5 to control to energize and de-energized of the electromagnet 5.

It should be noted that one or more electromagnets 5 and one or more permanent magnets 6 may be provided in the embodiment of the disclosure. The specific number of the electromagnets 5 and the specific number of the permanent magnets 6 may be set according to actual requirements, and are not limited in the embodiment of the disclosure.

It should be noted that in the embodiment of the disclosure, as shown in FIG. 1, it is preferable to fix the permanent magnet 6 to the cover 4 and fix the electromagnet 5 to the accommodation cavity 3, and the permanent magnet corresponds to the electromagnet in position, so as to facilitate the routing of the electromagnet 5.

It will be appreciated that in the embodiment of the disclosure, one of the electromagnet 5 and the permanent magnet 6 is fixed to the cover 4, the other is fixed to the accommodation cavity 3, and the electromagnet corresponds to the permanent magnet in position. Therefore, when the first controller 1 controls the electromagnet 5 to be separated from the permanent magnet 6, the accommodation cavity 3 may be separated from the cover 4 accordingly, such that the opening of the accommodation cavity 3 opens, and the user may operate the flow regulation valve 2 to control a flow. When the electromagnet 5 and the permanent magnet 6 are attracted to each other, it is possible to engage the accommodation cavity 3 with the cover 4 accordingly, such that the opening of the accommodation cavity 3 is closed, and the flow regulation valve 2 is enclosed inside the accommodation cavity 3.

It should be noted that in the embodiment of the disclosure, the cover 4 may be made of a transparent or translucent material, such that the user may know that the flow regulation valve 2 is disposed inside the accommodation cavity 3.

Specifically, in the embodiment of the disclosure, the operating state of the electronic flow control system of the anesthesia machine includes a failure state in which the first controller 1 controls to energize the electromagnet 5, such that the magnetism generated by a magnetic pole at an end of the electromagnet 5 close to the permanent magnet 6 is the same as the magnet generated by a magnetic pole at an end of the permanent magnet 6 close to the electromagnet 5 in order to separate the electromagnet 5 from the permanent magnet 6, and a working state in which the first controller controls to be de-energize the electromagnet 5.

It will be appreciated that in the embodiment of the disclosure, after the first controller 1 controls to de-energize the electromagnet 5, the user may manually operate to push the cover 4 toward the opening of the accommodation cavity 3, such that the permanent magnet 6 may magnetize the electromagnet 5 when the cove approaches at a certain distance from the accommodation cavity, allowing the electromagnet 5 and the permanent magnet 6 to attract each other so as to close the opening of the accommodation cavity 3.

Specifically, in the embodiment of the disclosure, the failure state of the electronic flow control system is at least one of: communication failure, valve failure, sensor failure, anesthesia machine power system failure and user interface failure of the electronic flow control system.

It should be noted that in the embodiment of the disclosure, the above-described failure states are a few exemplary failure states listed. Of course, in a practical application, the failure state may also include other failures that cause the electronic flow control system to fail to control a gas flow of the anesthesia machine, which are not limited in the embodiment of the disclosure.

It will be appreciated that in the embodiment of the disclosure, the working state of the electronic flow control system, namely, a state in which the electronic flow control system normally controls a gas flow of an anesthesia machine, excludes the related failures included in the above-described failure states.

It will be appreciated that in the case of the anesthesia machine using the electronic flow control system to control the gas flow, if the electronic flow control system is in a failure state, a relevant gas such as oxygen and air cannot be normally supplied to a patient, resulting in medical risks. Thus, in the embodiment of the disclosure, the backup flow control system, when the electronic flow control system fails, controls to turn on the backup ventilation mode in time for operating the flow regulation valve 2 by a user, ensuring the safety of the patient.

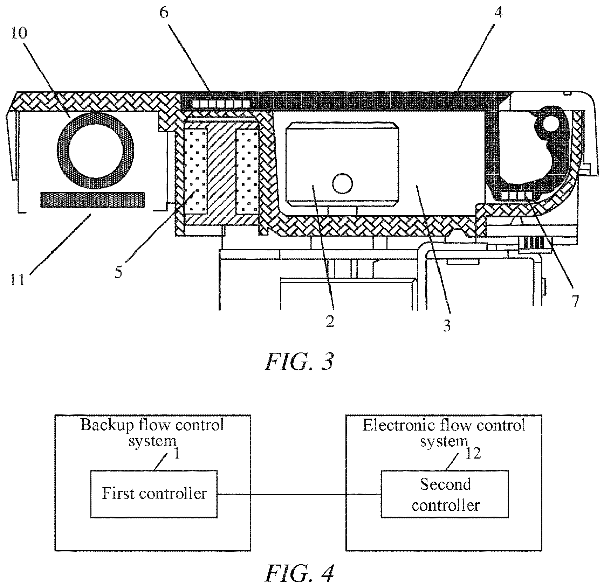

FIG. 3 is a third schematic structural diagram of an electronic flow control system according to an embodiment of the disclosure. As shown in FIGS. 2 and 3, the backup flow control system may further include a position sensor 7, where the position sensor 7 is connected to the first controller 1 and is fixed to the cover 4 or the accommodation cavity 3. The first controller 1 controls to turn on the backup ventilation mode when the position sensor 7 detects that the cover 4 is open.

It should be noted that in the embodiment of the disclosure, as shown in FIGS. 2 and 3, the position sensor 7 is fixed to the cover 4, of course, may be actually fixed to the accommodation cavity 3, which is not limited in the embodiment of the disclosure.

In particular, in the embodiment of the disclosure, the position sensor 7 is at least one of: a hall sensor, a photoelectric coupling sensor and a position switch. Of course, the position sensor 7 may also be other devices configured to achieve position detection, and may be selected according to actual requirements, which is not limited in the embodiment of the disclosure.

It should be noted that one or more position sensors 7 may be provided in the embodiment of the disclosure. With a plurality of position sensors 7, a problem that a single position sensor 7 fails and therefore the position of the cover 4 cannot be detected may be prevented. The specific number of the position sensors 7 may be selected according to actual requirements, and is not limited in the embodiment of the disclosure.

It will be appreciated that in the embodiment of the disclosure, the failure state such as the anesthesia machine power system failure or the user interface failure, for example, a failure such as a blank screen of a display screen, may be directly observed by the user, and therefore, the user may directly and manually open the cover 4 closing the accommodation cavity 3, the position sensor 7 thus detects that the cover 4 is in the opened position, and the user controls to turn on the backup ventilation mode and then can repair or replace relevant devices.

Specifically, in the embodiment of the disclosure, the first controller 1 is further configured to control to turn on the backup ventilation mode when detecting that the electromagnet 5 generates a separating induction current, and to control to turn off the backup ventilation mode when detecting that the electromagnet 5 generates an attracting induction current.

It will be appreciated that in the embodiment of the disclosure, the backup flow control system may also include no position sensor 7 for detecting the position of the cover 4. When the cover 4 is opened or closed, the electromagnet 5 will generate a corresponding induction current, such that whether the cover 4 is open or close may be determined, and the on or off of the backup ventilation mode may be controlled.

Specifically, in the embodiment of the disclosure, the flow regulation valve 2, when the backup ventilation mode is turned on, regulates a flow of a gas delivered to the patient according to a received regulation operation, and when the backup ventilation mode is turned off, stores a basic flow therein.

It should be noted that in the embodiment of the disclosure, the gas controlled by the flow regulation valve 2 is oxygen or oxygen and air. Specifically, when only one flow regulation valve 2 is disposed inside the accommodation cavity 6, control of oxygen is preferably applied to avoid hypoxia of the patient, and when a plurality of flow regulation valves 2 are disposed inside the accommodation cavity 3, respective control of flows of a plurality of types of gases may be achieved.

It will be appreciated that in the embodiment of the disclosure, the flow regulation valve 2 may store the basic flow when the backup ventilation mode is turned off, such that when the backup ventilation mode is controlled to be turned on next time, the basic flow may be supplied to the patient, avoiding the hypoxia of the patient due to the fact that the user does not operate the flow regulation valve 2 in time.

Specifically, in the embodiment of the disclosure, as shown in FIG. 2, the backup flow control system may further include a system switch 8, where the system switch 8 is connected to the flow regulation valve 2. The system switch 8, when in an OFF state, prevents leakage of the basic flow stored in the flow regulation valve 2.

It will be appreciated that in the embodiment of the disclosure, the flow regulation valve 2 is connected to the manually controlled system switch 8 such that the system switch 8 is manually switched off in the case of shutdown of the anesthesia machine, actually in the case where the electronic flow control system is in a failure state, to avoid leakage of the basic flow stored in the flow regulation valve 2. Of course, in the case of turning on the anesthesia machine, it is also necessary to manually switch on the system switch 8 such that the gas may be delivered to the patient when the backup ventilation mode is turned on.

Specifically, in the embodiment of the disclosure, as shown in FIG. 2, the backup flow control system may further include a flow sensor 9, where the flow sensor 9 is connected to the flow regulation valve 2 and the first controller 1 to detect the flow through the flow regulation valve 2 and to feedback the detected flow to the first controller 1.

It may be understood that in the embodiment of the disclosure, the flow sensor 9 may feedback the detected flow through the flow regulation valve 2 to the first controller 1, and when the backup ventilation mode is turned off and the electronic flow control system is activated for flow control, the first controller 1 may prompt the user to turn off the flow regulation valve 2 first, according to a detection result of the flow, avoiding a surge in flow when the backup ventilation mode is turned on next time.

It should be noted that in the embodiment of the disclosure, the flow regulation valve 2 may further be actually connected to a regulation valve switch, and whether the flow regulation valve 2 is turned off or not is detected by means of the regulation valve switch.

Specifically, in an embodiment of the disclosure, as shown in FIGS. 2 and 3, the backup flow control system may further include a glass tube flow meter 10, where the glass tube flow meter 10 is connected to the flow regulation valve 2 to display, when the backup ventilation mode is turned on, the gas flow regulated by the flow regulation valve 2.

It will be appreciated that in the embodiment of the disclosure, the glass tube flow meter 4 may display the gas flow regulated by the flow regulation valve 2 when the backup ventilation mode is turned on, such that the user can visually know whether an operation on the flow regulation valve 2 achieves expected flow control and make a corresponding adjustment.

Specifically, in the embodiment of the disclosure, as shown in FIGS. 2 and 3, the backup flow control system further includes a glass tube backlight 11, where the glass tube backlight 11 is installed on the back of the glass tube flow meter 10 and connected to the first controller 1. The first controller 1 controls the glass tube backlight 11 to emit light when controlling to turn on the backup ventilation mode.

It will be appreciated that in the embodiment of the disclosure, when the backup ventilation mode is turned on, the glass tube backlight 11 emits light, thereby achieving the effect of giving the user a prompt that the backup ventilation mode has been turned on.

It should be noted that in the embodiment of the disclosure, a power source for the electromagnet 5 and the glass tube backlight 11 is different from the power source for the electronic flow control system. Thus, the electromagnet 5 and the glass tube backlight 11 may operate normally even when a failure occurs to the power source of the electronic flow control system.

It should be noted that in embodiments of the disclosure, the operating state of the electronic flow control system may be identified by different devices.

In particular, in an embodiment of the disclosure, the electronic flow control system may include the first controller 1, namely, the first controller 1 is also a control device of the electronic flow control system, and the electronic flow control system shares one controller with the backup flow control system. Therefore, the first controller 1 may detect the operating state of the electronic flow control system.

It should be noted that in the embodiment of the disclosure, in a case where the electronic flow control system share the first controller 1 with the backup flow control system, when the first controller 1 fails, the electromagnet 5 is in an energized state by default and separated from the permanent magnet 6, thereby controlling to open the cover 4.

FIG. 4 is a first schematic diagram of a connection between an exemplary backup flow control system and an electronic flow control system according to an embodiment of the disclosure. As shown in FIG. 4, in the embodiment of the disclosure, the electronic flow control system includes a second controller 12, where the first controller 1 is connected to the second controller 12. The second controller 12 detects an operating state of the electronic flow control system and transmits the detected operating state to the first controller 1.

It will be appreciated that in the embodiment of the disclosure, in a case where the electronic flow control system and the backup flow control system use different controllers, the second controller 12 of the electronic flow control system may achieve detection of the operating state of the electronic flow control system, thereby transmitting the detected operating state to the first controller 1 of the backup electronic flow control system for the first controller 1 to achieve control of opening and closing of the cover 4.

It should be noted that in the embodiment of the disclosure, the first controller 1 determines that the operating state of the electronic flow control system is a failure state when the interruption of communication with the second controller 12 is detected.

It will be appreciated that in the embodiment of the disclosure, if the second controller 12 of the electronic flow control system itself fails, the communication between the first controller 1 and the second controller 12 will be interrupted, and thus the first controller 1 may determine that the operating state of the electronic flow control system is the failure state.

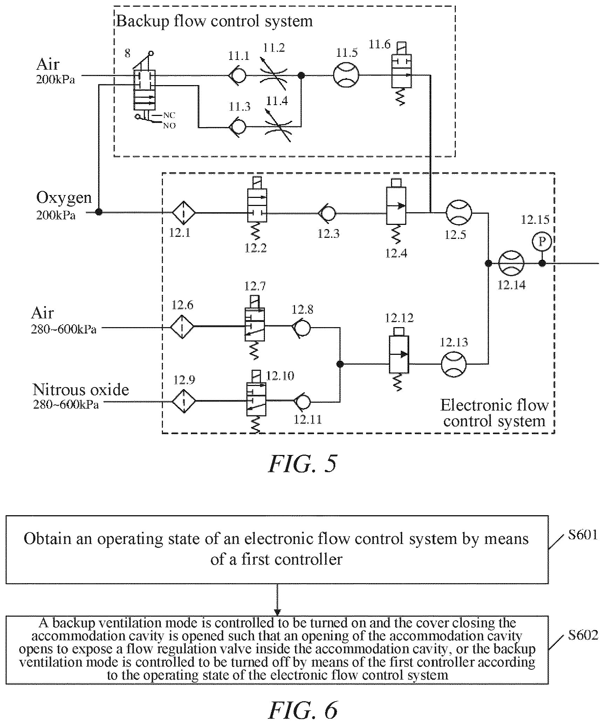

FIG. 5 is a second schematic diagram of a connection between an exemplary backup flow control system and an electronic flow control system according to an embodiment of the disclosure. As shown in FIG. 5, the backup flow control system may include: a system switch 8, an one-way valve 11.1, an oxygen needle valve 11.2, an one-way valve 11.3, an air needle valve 11.4, a float flow meter 11.5, and a normally open switch valve 11.6. The oxygen needle valve 11.2 and the air needle valve 11.4 act as two flow regulation valves 1, such that when the backup ventilation mode is turned on, flow control of oxygen and air is correspondingly achieved. The float flow meter 11.5 is the above-mentioned glass tube flow meter 10, and displays a gas flow when the backup ventilation mode is turned on. The normally open switch valve 11.6 is opened when the backup ventilation mode is turned on. The electronic flow control system includes: a filter 12.1, a normally closed switch valve 12.2, an one-way valve 12.3, a proportional valve 12.4, a flow sensor 12.5, a filter 12.6, a 2/3 switching valve 12.7, an one-way valve 12.8, a filter 12.9, a 2/3 switching valve 12.10, an one-way valve 12.11, a proportional valve 12.12, a flow sensor 12.13, a flow sensor 12.14 and a pressure sensor 12.15. The normally closed switch valve 12.2 is closed when the backup ventilation mode is turned on, such that oxygen enters the backup electronic flow control system to achieve flow control. In a case where the operating state of the electronic flow control system is a working state, a process during which the above-described devices included in the electronic flow control system cooperatively operate to achieve normal oxygen delivery to the patient is known in the prior art, and will not be described in detail herein.

An embodiment of the disclosure provides a backup flow control system applicable to an anesthesia machine. The backup flow control system turns on a backup ventilation mode when an electronic flow control system of the anesthesia machine has failed. The backup flow control system includes: a first controller, a flow regulation valve, an accommodation cavity and a cover, where the accommodation cavity is a hollow structure having an opening, the cover is connected to the opening of the accommodation cavity to close the opening, and the flow regulation valve is disposed inside the accommodation cavity; and the first controller, according to an operating state of the electronic flow control system, controls the backup ventilation mode to be turned on and the cover is opened such that the opening opens to expose the flow regulation valve, or controls the backup ventilation mode to be turned off. The backup flow control system provided by the embodiment of the disclosure not only protects the flow regulation valve from accidental regulation by means of the openable cover, but also has a simple structure, and is capable of automatically opening the cover when backup ventilation is required, thereby improving flexibility and reliability.

An embodiment of the disclosure provides a backup flow control method applicable to an anesthesia machine, the anesthesia machine including an electronic flow control system. FIG. 6 is a schematic flow diagram of a backup flow control method applicable to an anesthesia machine according to an embodiment of the disclosure. As shown in FIG. 6, the method mainly includes the following steps.

At step S601, an operating state of the electronic flow control system is obtained by means of a first controller.

In the embodiments of the disclosure, referring to FIGS. 1 to 4, the backup flow control system obtains the operating state of the electronic flow control system by means of the first control 1.

It should be noted that in the embodiments of the disclosure, the operating state of the electronic flow control system include a failure state and a working state. The failure state is at least one of: communication failure, valve failure, sensor failure, anesthesia machine power system failure and user interface failure of the electronic flow control system. Of course, in a practical application, the failure state may also include other failures that cause the electronic flow control system to fail to control a gas flow of the anesthesia machine, which are not limited in the embodiment of the disclosure. The working state is a state in which the electronic flow control system normally achieves the control of the gas flow of the anesthesia machine, and excludes the related failures included in the above-described failure states.

It should be noted that in the embodiments of the disclosure, the backup flow control system obtaining, when the first controller 1 is further included in the electronic flow control system, an operating state of the electronic flow control system by means of the first controller 1 includes: detecting the operating state of the electronic flow control system operating state by means of the first controller 1.

It should be noted that in the embodiments of the disclosure, in a case where the controllers of the electronic flow control system and the backup flow control system are different, namely, the electronic flow control system includes no first controller 1, an interaction between the electronic flow control system and the backup flow control system may be possible, such that the operating state of the electronic flow control system is obtained by mean of the first controller 1.

Specifically, in the embodiments of the disclosure, the electronic flow control system includes the second controller 12, and the first controller 1 is connected to the second controller 12. The backup flow control system obtaining an operating state of the electronic flow control system by means of the first controller 1 includes: detecting the operating state of the electronic flow control system by means of the second controller 12; and receiving the operating state detected by the second controller 12 by means of the first controller 1.

It should be noted that in the embodiments of the disclosure, in a case where the electronic flow control system includes the second controller 12 and the first controller 1 obtains the operating state of the electronic flow control system by means of the second controller 12, the first controller 1 obtaining an operating state of the electronic flow control system further includes: determining, by means of the first controller 1, that the operating state of the electronic flow control system is a failure state when the interruption of communication with the second controller 12 is detected.

It will be appreciated that in the embodiments of the disclosure, if the second controller 12 itself fails, normal control of the gas flow by the electronic flow control system cannot be achieved, the communication between the first controller 1 and the second controller 12 will also be interrupted, and in this case, the first controller 1 may directly determine that the operating state of the electronic flow control system is the failure state.

At step S602, the backup ventilation mode is controlled to be turned on and the cover closing the accommodation cavity is opened such that the opening of the accommodation cavity opens to expose the flow regulation valve inside the accommodation cavity, or the backup ventilation mode is controlled to be turned off by means of the first controller according to the operating state of the electronic flow control system.

In the embodiments of the disclosure, referring to FIGS. 1 to 4, after obtaining the operating state of the electronic flow control system, the backup flow control system controls the backup ventilation mode to be turned on or off by means of the first controller 1 according to the operating state of the electronic flow control system.

Specifically, in the embodiments of the disclosure, controlling the backup ventilation mode to be turned on and opening the cover 4 closing the accommodation cavity 3 or controlling the backup ventilation mode to be turned off and closing the cover 4 by means of the first controller 1 according to the operating state of the electronic flow control system includes: controlling, by means of the first controller 1, energization and de-energization of the electromagnet 5 according to the operating state of the electronic flow control system, where one of the permanent magnet 6 and the electromagnet 5 is fixed to the cover 4, the other one thereof is fixed to the accommodation cavity 3, the permanent magnet 6 corresponds to the electromagnet 5 in position, and the first controller 1 is connected to the electromagnet 5.

It should be noted that in the embodiments of the disclosure, the operating state of the electronic flow control system include a failure state and a working state, and controlling, by means of the first controller 1, energization and de-energization of the electromagnet 5 according to the operating state of the electronic flow control system includes: by means of the first controller 1, controlling the electromagnet 5 to be energized when the electronic flow control system is in the failure state, such that the magnetism generated by a magnetic pole at an end of the electromagnet 5 close to the permanent magnet 6 is the same as the magnetism generated by a magnetic pole at an end of the permanent magnet 6 close to the electromagnet 5 in order to separate the electromagnet 5 from the permanent magnet 6, and controlling the electromagnet 5 to be de-energized when the electronic flow control system is in the working state.

It should be noted that in embodiments of the disclosure, the backup ventilation mode may also be turned on by means of an manual operation by the user.

Specifically, in the embodiment of the disclosure, the backup flow control system may control, by means of the first controller 1, the backup ventilation mode to be turned on when the position sensor 7 detects that the cover is in an opened position;
or may control, by means of the first controller 1, the backup ventilation mode to be turned on when it is detected that the electromagnet 5 generates a separating induction current, and control the backup ventilation mode to be turned off when it is detected that the electromagnet 5 generates an attracting induction current.

It will be appreciated that in the disclosure, during practical application, in some cases, such as a blank screen problem of the anesthesia machine, it is also required to use the backup flow control system so as to repair and replace relevant devices of the anesthesia machine, and such a problem may be directly observed by a user. Therefore, the user may manually use the backup flow control system, namely, manually operate the backup flow control system to open the cover 4 closing the accommodation cavity 3 and turn on the backup ventilation mode.

It will be appreciated that in the embodiment of the disclosure, for a case where the user manually opens the cover 4 closing the accommodation cavity 3, the first controller 1 may achieve detection of a position of the cover 4 by means of the position sensor 7 fixed to the cover 4 or to the accommodation cavity 3, thereby controlling the backup ventilation mode to be turned on when it is detected that the cover 4 is in the opened position. Furthermore, when the cover 4 is opened or closed, the electromagnet 5 generates a corresponding induction current, and the first controller 1 may determine that the cover 4 is in the opened position or the closed position by detecting the induction current, so as to turn on or off the backup ventilation mode.

It should be noted that in the embodiments of the disclosure, the backup flow control system may further include the system switch 8, the flow sensor 9, the glass tube flow meter 10, and the glass tube backlight 11. The user may manually switch off the system switch 8 for no leakage of the basic flow stored in the flow regulation valve 2 in the case of shutdown of the anesthesia machine. The flow sensor 9 may detect a flow through the flow regulation valve 2 and feed back the detected flow to the first controller 1. The glass tube flow meter 10 may display a gas flow regulated by the flow regulation valve 4 when the backup ventilation mode is turned on. The first controller 1 may control the glass tube backlight 11 to emit light when controlling the backup ventilation mode to be turned on, which gives a prompt that the backup ventilation mode is turned on.

An embodiment of the disclosure provides a backup flow control method applicable to an anesthesia machine, the anesthesia machine including an electronic flow control system, the method including: obtaining an operating state of the electronic flow control system by means of a first controller; and controlling a backup ventilation mode to be turned on and opening a cover closing an accommodation cavity such that an opening of the accommodation cavity opens to expose a flow regulation valve inside the accommodation cavity, or controlling the backup ventilation mode to be turned off by means of the first controller according to the operating state of the electronic flow control system. By means of the backup flow control method provided by the embodiments of the disclosure, not only the flow regulation valve is protected from accidental regulation by means of the openable cover, but also a simple structure is achieved, and the cover may be automatically opened when backup ventilation is required, thereby improving flexibility and reliability.

The above descriptions are merely specific embodiments of the disclosure. The scope of protection of the disclosure shall be subject to the scope of protection of the claims.

### INDUSTRIAL APPLICABILITY

An embodiment of the disclosure provide a backup flow control system applicable to an anesthesia machine. The backup flow control system turns on a backup ventilation mode when an electronic flow control system of the anesthesia machine has failed. The backup flow control system includes: a first controller, a flow regulation valve, an accommodation cavity and a cover, where the accommodation cavity is a hollow structure having an opening, the cover is connected to the opening of the accommodation cavity to close the opening, and the flow regulation valve is disposed inside the accommodation cavity; and the first controller, according to an operating state of the electronic flow control system, controls to turn on the backup ventilation mode and to open the cover, such that the opening opens to expose the flow regulation valve, or controls to turn off the backup ventilation mode. The backup flow control system provided by the embodiment of the disclosure not only protects the flow regulation valve from accidental regulation by the openable cover, but also has a simple structure, and is capable of automatically opening the cover when backup ventilation is required, thereby improving flexibility and reliability.

## Claims

1. A backup flow control system applicable to an anesthesia machine,
wherein the backup flow control system is connected with an electronic flow control system of the anesthesia machine and configured to turn on a backup ventilation mode when the electronic flow control system of the anesthesia machine has failed, and the backup flow control system comprises: a first controller (1), a flow regulation valve (2), an accommodation cavity (3) and a cover (4), wherein
the accommodation cavity (3) is a hollow structure having an opening, the cover (4) is connected to the opening of the accommodation cavity (3) to close the opening, and the flow regulation valve (2) is disposed inside the accommodation cavity (3); and
the first controller (1) controls to turn on the backup ventilation mode and to open the cover (4), such that the opening opens to expose the flow regulation valve (2) when the electronic flow control system is in a failure state; or controls to turn off the backup ventilation mode when the electronic flow control system is in a working state.

2. The system of claim 1, wherein
the system further comprises an electromagnet (5) and a permanent magnet (6), wherein power sources for the electromagnet (5) and the electronic flow control system are different, one of the permanent magnet (6) and the electromagnet (5) is fixed to the cover (4) and the other one thereof is fixed to the accommodation cavity (3), and the permanent magnet (6) corresponds to the electromagnet (5) in position; and the first controller (1) is connected to the electromagnet (5) and controls to energize and de-energize the electromagnet (5).

3. The system of claim 2, wherein
the first controller (1) controls to energize the electromagnet (5) when the electronic flow control system is in the failure state, such that magnetism generated by a magnetic pole at an end of the electromagnet (5) close to the permanent magnet (6) is the same as magnetism generated by a magnetic pole at an end of the permanent magnet (6) close to the electromagnet (5), in order to separate the electromagnet (5) from the permanent magnet (6), and controls to de-energize the electromagnet (5) when the electronic flow control system is in the working state,
preferably the failure state is at least one of: communication failure, valve failure, sensor failure, anesthesia machine power system failure and user interface failure of the electronic flow control system.

4. The system of claim 1, wherein the system further comprises a position sensor (7),
wherein the position sensor (7) is connected to the first controller (1) and fixed to the cover (4) or the accommodation cavity (3); and
the first controller (1) controls to turn on the backup ventilation mode when the position sensor (7) detects that the cover (4) is opened,
preferably the position sensor (7) is at least one of: a hall sensor, a photoelectric coupling sensor and a position switch.

5. The system of claim 2, wherein
the first controller (1) controls to turn on the backup ventilation mode when it is detected that the electromagnet (5) generates a separating induction current, and controls to turn off the backup ventilation mode when it is detected that the electromagnet (5) generates an attracting induction current.

6. The system of claim 1, wherein
the flow regulation valve (2) regulates a flow of a gas delivered to a patient according to a received regulation operation, when the backup ventilation mode is turned on, and stores a basic flow therein, when the backup ventilation mode is turned off, preferably the gas is oxygen, or oxygen and air, and preferably the system further comprises a system switch (8), wherein the system switch (8) is connected to the flow regulation valve (2), and the system switch (8) prevents leakage of the basic flow stored in the flow regulation valve (2), when the system switch (8) is closed.

7. The system of claim 1, wherein:
the system further comprises a flow sensor (9), wherein the flow sensor (9) is connected to the flow regulation valve (2) and the first controller (1), so as to detect a flow through the flow regulation valve (2) and to feedback the detected flow to the first controller (1); or
the system further comprises a glass tube flow meter (10), wherein the glass tube flow meter (10) is connected to the flow regulation valve (2), so as to display a gas flow regulated by the flow regulation valve (2), when the backup ventilation mode is turned on, preferably the system further comprises a glass tube backlight (11), wherein the glass tube backlight (11) is disposed at a back of the glass tube flow meter (10) and is connected to the first controller (1), and the first controller (1) controls the glass tube backlight (11) to emit light when controlling to turn on the backup ventilation mode, and preferably a power source for the glass tube backlight (11) is different from a power source for the electronic flow control system.

8. The system of claim 1, wherein:
the first controller (1) is also included in the electronic flow control system, and the first controller (1) detects an operating state of the electronic flow control system; or
the electronic flow control system comprises a second controller (12), wherein the first controller (1) is connected to the second controller (12); and the second controller (12) detects an operating state of the electronic flow control system and transmits the detected operating state to the first controller (1), preferably the first controller (1) determines that the operating state of the electronic flow control system is the failure state, when interruption of communication with the second controller (12) is detected.

9. The system of claim 2, wherein,
when the first controller (1) fails, the electromagnet (5) is energized and separated from the permanent magnet (6), thereby controlling to open the cover (4).

10. A backup flow control method applicable to an anesthesia machine, executed by a backup flow control system comprising a first controller, a flow regulation valve, an accommodation cavity and a cover, the backup flow control system being connected with an electronic flow control system of the anesthesia machine, wherein the method comprises:
obtaining (S601) an operating state of the electronic flow control system by the first controller, the operating state of the electronic flow control system comprising a failure state and a working state; and
controlling (S602), by the first controller, to turn on a backup ventilation mode and to open the cover that closes the accommodation cavity, such that an opening of the accommodation cavity opens to expose the flow regulation valve inside the accommodation cavity, when the electronic flow control system is in the failure state, or controlling, by the first controller, to turn off the backup ventilation mode when the electronic flow control system is in the working state.

11. The method of claim 10, wherein controlling, by the first controller, to turn on the backup ventilation mode and to open the cover that closes the accommodation cavity, when the electronic flow control system is in the failure state, or controlling, by the first controller, to turn off the backup ventilation mode when the electronic flow control system is in the working state comprises:
controlling, by the first controller, to energize and de-energize an electromagnet according to the operating state of the electronic flow control system;
wherein one of a permanent magnet and the electromagnet is fixed to the cover, the other one thereof is fixed to the accommodation cavity, the permanent magnet corresponds to the electromagnet in position, and the first controller is connected to the electromagnet.

12. The method of claim 11, wherein controlling, by the first controller, to energize and de-energize the electromagnet according to the operating state of the electronic flow control system comprises:
controlling, by the first controller, to energize the electromagnet when the electronic flow control system is in the failure state, such that magnetism generated by a magnetic pole at an end of the electromagnet close to the permanent magnet is the same as magnetism generated by a magnetic pole at an end of the permanent magnet close to the electromagnet, in order to separate the electromagnet from the permanent magnet, and controlling, by the first controller, to de-energize the electromagnet when the electronic flow control system is in the working state.

13. The method of claim 10, wherein the method further comprises:
controlling, by the first controller, to turn on the backup ventilation mode when a position sensor detects that the cover is opened, preferably controlling, by the first controller, to turn on the backup ventilation mode when it is detected that the electromagnet generates a separating induction current, and controlling, by the first controller, to turn off the backup ventilation mode when it is detected that the electromagnet generates an attracting induction current.

14. The method of claim 10, wherein, if the first controller is also included in the electronic flow control system, obtaining the operating state of the electronic flow control system by the first controller comprises:
detecting the operating state of the electronic flow control system by the first controller.

15. The method of claim 10, wherein the electronic flow control system comprises a second controller, the first controller being connected to the second controller, and obtaining the operating state of the electronic flow control system by the first controller comprises:
detecting the operating state of the electronic flow control system by the second controller; and receiving, by the first controller, the operating state detected by the second controller, preferably obtaining the operating state of the electronic flow control system by the first controller further comprises:
determining, by the first controller, that the operating state of the electronic flow control system is the failure state when interruption of communication with the second controller is detected.

## Patentansprüche

1. Ein Backup-Durchflusssteuerungssystem für ein Anästhesiegerät,
wobei das Backup-Durchflusssteuerungssystem mit einem elektronischen Durchflusssteuerungssystem des Anästhesiegeräts verbunden und so konfiguriert ist, dass es einen Backup-Beatmungsmodus einschaltet, wenn das elektronische Durchflusssteuerungssystem des Anästhesiegeräts ausgefallen ist, und das Backup-Durchflusssteuerungssystem umfasst: eine erste Steuereinheit (1), ein Durchflussregulierungsventil (2), einen Aufnahmehohlraum (3) und eine Abdeckung (4), wobei
der Aufnahmehohlraum (3) eine hohle Struktur mit einer Öffnung ist, die Abdeckung (4) mit der Öffnung des Aufnahmehohlraums (3) verbunden ist, um die Öffnung zu schließen, und das Durchflussregulierungsventil (2) innerhalb des Aufnahmehohlraums (3) angeordnet ist; und
das erste Steuereinheit (1) steuert das Einschalten des Reservelüftungsmodus und das Öffnen der Abdeckung (4), so dass sich die Öffnung öffnet, um das Durchflussregulierungsventil (2) freizulegen, wenn sich das elektronische Durchflusssteuerungssystem in einem Fehlerzustand befindet; oder steuert das Ausschalten des Reservelüftungsmodus, wenn sich das elektronische Durchflusssteuerungssystem in einem Arbeitszustand befindet.

2. Das System nach Anspruch 1, wobei das System ferner Folgendes umfasst
einen Elektromagneten (5) und einen Dauermagneten (6), wobei die Stromquellen für den Elektromagneten (5) und das elektronische Durchflusssteuerungssystem unterschiedlich sind, einem von dem Dauermagneten (6) und dem Elektromagneten (5) an der Abdeckung (4) und der andere davon an dem Aufnahmehohlraum (3) befestigt ist und der Dauermagnet (6) in seiner Position dem Elektromagneten (5) entspricht; und die erste Steuereinheit (1) mit dem Elektromagneten (5) verbunden ist und steuert, um den Elektromagneten (5) ein- und ausschalten.

3. Das System nach Anspruch 2, wobei
der erste Steuereinheit (1) steuert, um den Elektromagneten (5) zu erregen, wenn das elektronische Durchflusssteuerungssystem im Fehlerzustand ist, so dass der Magnetismus, der von einem Magnetpol an einem Ende des Elektromagneten (5) nahe dem Dauermagneten (6) erzeugt wird, der gleiche ist wie der Magnetismus, der von einem Magnetpol an einem Ende des Dauermagneten (6) nahe dem Elektromagneten (5) erzeugt wird, um den Elektromagneten (5) von dem Dauermagneten (6) zu trennen, und steuert, um den Elektromagneten (5) stromlos zu machen, wenn sich das elektronische Durchflusssteuerungssystem im Arbeitszustand befindet, wobei der Fehlerzustand vorzugsweise mindestens einer der folgenden ist: Kommunikationsausfall, Ventilausfall, Sensorausfall, Ausfall des Stromversorgungssystems des Anästhesiegeräts und Ausfall der Benutzerschnittstelle des elektronischen Durchflusssteuerungssystem s.

4. System nach Anspruch 1, wobei das System ferner einen Positionssensor (7) umfasst,
wobei der Positionssensor (7) mit dem ersten Steuereinheit (1) verbunden und an der Abdeckung (4) oder dem Aufnahmehohlraum (3) befestigt ist; und
das erste Steuereinheit (1) steuert das Einschalten des Notlüftungsmodus, wenn der Positionssensor (7) feststellt, dass die Abdeckung (4) geöffnet ist, wobei der Positionssensor (7) vorzugsweise mindestens einer der folgenden ist: ein Hall-Sensor, ein photoelektrischer Kopplungssensor und ein Positionsschalter.

5. Das System nach Anspruch 2, wobei
die erste Steuereinheit (1) steuert das Einschalten des Notlüftungsmodus, wenn festgestellt wird, dass der Elektromagnet (5) einen trennenden Induktionsstrom erzeugt, und steuert das Ausschalten des Notlüftungsmodus, wenn festgestellt wird, dass der Elektromagnet (5) einen anziehenden Induktionsstrom erzeugt.

6. Das System nach Anspruch 1, wobei
das Durchflussregulierungsventil (2) einen Fluss eines einem Patienten zugeführten Gases gemäß einer empfangenen Regulierungsoperation reguliert, wenn der Ersatzbeatmungsmodus eingeschaltet ist, und einen Basisfluss darin speichert, wenn der Ersatzbeatmungsmodus ausgeschaltet ist, vorzugsweise ist das Gas Sauerstoff oder Sauerstoff und Luft, und vorzugsweise umfasst das System ferner einen Systemschalter (8), wobei der Systemschalter (8) mit dem Durchflussregulierungsventil (2) verbunden ist und der Systemschalter (8) ein Auslaufen des in dem Durchflussregulierungsventil (2) gespeicherten Basisflusses verhindert, wenn der Systemschalter (8) geschlossen ist.

7. Das System nach Anspruch 1, wobei:
das System ferner einen Durchflusssensor (9) umfasst, wobei der Durchflusssensor (9) mit dem Durchflussregulierungsventil (2) und dem ersten Regler (1) verbunden ist, um einen Durchfluss durch das Durchflussregulierungsventil (2) zu erfassen und den erfassten Durchfluss an den ersten Regler (1) rückzukoppeln; oder
das System ferner einen Glasrohr-Durchflussmesser (10) umfasst, wobei der Glasrohr-Durchflussmesser (10) mit dem Durchflussregulierungsventil (2) verbunden ist, um einen durch das Durchflussregulierungsventil (2) regulierten Gasfluss anzuzeigen, wenn der Ersatzbeatmungsmodus eingeschaltet ist, wobei das System vorzugsweise ferner eine Glasrohr-Hintergrundbeleuchtung (11) umfasst, wobei die Glasrohr-Hintergrundbeleuchtung (11) an einer Rückseite des Glasrohr-Durchflussmessers (10) angeordnet und mit der ersten Steuereinheit (1) verbunden ist, und die erste Steuereinheit (1) die Glasrohr-Hintergrundbeleuchtung (11) so steuert, dass sie Licht ausstrahlt, wenn sie das Einschalten des Ersatzbeatmungsmodus steuert, und vorzugsweise eine Energiequelle für die Glasrohr-Hintergrundbeleuchtung (11) von einer Energiequelle für das elektronische Durchflusssteuerungssystem verschieden ist.

8. Das System nach Anspruch 1, wobei:
das erste Steuereinheit (1) ebenfalls in dem elektronischen Durchflusssteuerungssystem enthalten ist und das erste Steuereinheit (1) einen Betriebszustand des elektronischen Durchflusssteuerungssystems erfasst; oder
das elektronische Durchflusssteuerungssystem einen zweiten Steuereinheit (12) umfasst, wobei der erste Steuereinheit (1) mit dem zweiten Steuereinheit (12) verbunden ist; und der zweite Steuereinheit (12) einen Betriebszustand des elektronischen Durchflusssteuerungssystems erfasst und den erfassten Betriebszustand an den ersten Steuereinheit (1) übermittelt, wobei vorzugsweise der erste Steuereinheit (1) feststellt, dass der Betriebszustand des elektronischen Durchflusssteuerungssystems der Ausfallzustand ist, wenn eine Unterbrechung der Kommunikation mit dem zweiten Steuereinheit (12) erfasst wird.

9. Das System nach Anspruch 2, wobei,
wenn das erste Steuereinheit (1) ausfällt, wird der Elektromagnet (5) erregt und vom Dauermagneten (6) getrennt, wodurch die Öffnung der Abdeckung (4) gesteuert wird.

10. Backup-Durchflusssteuerungsverfahren, das auf ein Anästhesiegerät anwendbar ist und von einem Backup-Durchflusssteuerungssystem ausgeführt wird, das eine erste Steuerung, ein Durchflussregelungsventil, einen Aufnahmehohlraum und eine Abdeckung umfasst, wobei das Backup-Durchflusssteuerungssystem mit einem elektronischen Durchflusssteuerungssystem des Anästhesiegeräts verbunden ist, wobei das Verfahren umfasst:
Ermitteln (S601) eines Betriebszustands des elektronischen Durchflusssteuerungssystems durch den ersten Controller, wobei der Betriebszustand des elektronischen Durchflusssteuerungssystems einen Fehlerzustand und einen Arbeitszustand umfasst; und
Steuerung (S602) durch die erste Steuereinheit, um einen Ersatzbelüftungsmodus einzuschalten und die Abdeckung zu öffnen, die den Aufnahmehohlraum verschließt, so dass sich eine Öffnung des Aufnahmehohlraums öffnet, um das Durchflussregulierungsventil innerhalb des Aufnahmehohlraums freizulegen, wenn sich das elektronische Durchflusssteuerungssystem im Fehlerzustand befindet, oder Steuerung durch die erste Steuereinheit, um den Ersatzbelüftungsmodus auszuschalten, wenn sich das elektronische Durchflusssteuerungssystem im Arbeitszustand befindet.

11. Verfahren nach Anspruch 10, wobei die Steuerung durch die erste Steuereinheit das Einschalten des Notlüftungsmodus und das Öffnen der Abdeckung, die den Aufnahmehohlraum verschließt, wenn sich das elektronische Durchflusssteuerungssystem im Fehlerzustand befindet, oder die Steuerung durch die erste Steuereinheit das Ausschalten des Notlüftungsmodus, wenn sich das elektronische Durchflusssteuerungssystem im Arbeitszustand befindet, umfasst:
Steuerung der Aktivierung und Deaktivierung eines Elektromagneten durch das erste Steuereinheit in Abhängigkeit vom Betriebszustand des elektronischen Durchflusssteuerungssystems;
wobei einer von einem Dauermagneten und dem Elektromagneten an der Abdeckung befestigt ist, der andere davon an dem Aufnahmehohlraum befestigt ist, der Dauermagnet in seiner Position dem Elektromagneten entspricht und das erste Steuereinheit mit dem Elektromagneten verbunden ist.

12. Verfahren nach Anspruch 11, wobei die Steuerung durch die erste Steuereinheit zum Ein- und Ausschalten des Elektromagneten entsprechend dem Betriebszustand des elektronischen Durchflusssteuerungssystems umfasst:
Steuereinheit durch die erste Steuereinheit, um den Elektromagneten zu erregen, wenn sich das elektronische Durchflusssteuerungssystem im Fehlerzustand befindet, so dass der von einem Magnetpol an einem Ende des Elektromagneten in der Nähe des Dauermagneten erzeugte Magnetismus der gleiche ist wie der von einem Magnetpol an einem Ende des Dauermagneten in der Nähe des Elektromagneten erzeugte Magnetismus, um den Elektromagneten von dem Dauermagneten zu trennen, und Steuerung durch die erste Steuereinheit, um den Elektromagneten abzuschalten, wenn sich das elektronische Durchflusssteuerungssystem im Arbeitszustand befindet.

13. Verfahren nach Anspruch 10, wobei das Verfahren ferner umfasst:
Steuerung durch die erste Steuereinheit, um den Notlüftungsmodus einzuschalten, wenn ein Positionssensor feststellt, dass die Abdeckung geöffnet ist, vorzugsweise Steuerung durch die erste Steuereinheit, um den Notlüftungsmodus einzuschalten, wenn festgestellt wird, dass der Elektromagnet einen trennenden Induktionsstrom erzeugt, und Steuerung durch die erste Steuereinheit, um den Notlüftungsmodus auszuschalten, wenn festgestellt wird, dass der Elektromagnet einen anziehenden Induktionsstrom erzeugt.

14. Verfahren nach Anspruch 10, wobei, wenn die erste Steuereinheit ebenfalls in dem elektronischen Durchflusssteuerungssystem enthalten ist, das Ermitteln des Betriebszustands des elektronischen Durchflusssteuerungssystems durch die erste Steuereinheit umfasst:
Erfassen des Betriebszustands des elektronischen Durchflusssteuerungssystems durch die erste Steuereinheit.

15. Verfahren nach Anspruch 10, wobei das System eine zweite Steuereinheit umfasst, wobei die erste Steuereinheit mit der zweiten Steuereinheit verbunden ist, und das Erhalten des Betriebszustands des elektronischen Durchflusssteuerungssystems durch die erste Steuereinheit besteht:
Erfassen des Betriebszustands des elektronischen Durchflusssteuerungssystems durch die zweite Steuereinheit; und Empfangen des von der zweiten Steuereinheit erfassten Betriebszustands durch die erste Steuereinheit, wobei das Erhalten des Betriebszustands des elektronischen Durchflusssteuerungssystems durch die erste Steuereinheit vorzugsweise weiterhin umfasst:
Feststellung durch die erste Steuereinheit, dass der Betriebszustand des elektronischen Durchflusssteuerungssystems der Fehlerzustand ist, wenn eine Unterbrechung der Kommunikation mit dem zweiten Steuereinheit festgestellt wird.

## Revendications

1. Système de secours de commande d'écoulement applicable à une machine d'anesthésie, où
le système de secours de commande d'écoulement est relié à un système de commande d'écoulement électronique de la machine d'anesthésie et configuré pour activer un mode de ventilation de secours lorsque le système de commande d'écoulement électronique de la machine d'anesthésie a connu une défaillance, et le système de secours de commande d'écoulement comprend : une première unité de commande (1), une vanne de régulation d'écoulement (2), une cavité de réception (3) et un couvercle (4), où
la cavité de réception (3) est une structure creuse présentant une ouverture, le couvercle (4) est relié à l'ouverture de la cavité de réception (3) pour fermer l'ouverture, et la vanne de régulation d'écoulement (2) est disposée à l'intérieur de la cavité de réception (3) ; et
la première unité de commande (1) ordonne d'activer le mode de ventilation de secours et d'ouvrir le couvercle (4), de telle sorte que l'ouverture est ouverte pour exposer la vanne de régulation d'écoulement (2) lorsque le système de commande d'écoulement électronique se trouve dans un état de défaillance ; ou ordonne de désactiver le mode de ventilation de secours lorsque le système de commande d'écoulement électronique se trouve dans un état de bon fonctionnement.

2. Système selon la revendication 1, où
le système comprend en outre un électroaimant (5) et un aimant permanent (6), où des sources d'alimentation pour l'électroaimant (5) et le système de commande d'écoulement électronique sont différentes, un premier aimant parmi l'aimant permanent (6) et l'électroaimant (5) est fixé sur le couvercle (4) et l'autre aimant est fixé sur la cavité de réception (3), et l'aimant permanent (6) correspond à l'électroaimant (5) en termes de position ; et la première unité de commande (1) est reliée à l'électroaimant (5) et ordonne d'exciter et de désexciter l'électroaimant (5).

3. Système selon la revendication 2, dans lequel
la première unité de commande (1) ordonne d'exciter l'électroaimant (5) lorsque le système de commande d'écoulement électronique se trouve dans l'état de défaillance, de telle sorte qu'un champ magnétique généré par un pôle magnétique à une extrémité de l'électroaimant (5) proche de l'aimant permanent (6) est identique à un champ magnétique généré par un pôle magnétique à une extrémité de l'aimant permanent (6) proche de l'électroaimant (5), de sorte à séparer l'électroaimant (5) de l'aimant permanent (6), et ordonne de désexciter l'électroaimant (5) lorsque le système de commande d'écoulement électronique se trouve dans l'état de bon fonctionnement,
de préférence l'état de défaillance est au moins une défaillance parmi : une défaillance de communication, une défaillance de vanne, une défaillance de capteur, une défaillance de système d'alimentation de machine d'anesthésie et une défaillance d'interface utilisateur du système de commande d'écoulement électronique.

4. Système selon la revendication 1, où
le système comprend en outre un capteur de position (7),
où le capteur de position (7) est relié à la première unité de commande (1) et fixé sur le couvercle (4) ou la cavité de réception (3) ; et
la première unité de commande (1) ordonne d'activer le mode de ventilation de secours lorsque le capteur de position (7) détecte que le couvercle (4) est ouvert,
de préférence le capteur de position (7) est au moins un capteur parmi : un capteur à effet Hall, un capteur de couplage photoélectrique et un commutateur de position.

5. Système selon la revendication 2, dans lequel
la première unité de commande (1) ordonne d'activer le mode de ventilation de secours lorsqu'il est détecté que l'électroaimant (5) génère un courant d'induction séparatif, et ordonne de désactiver le mode de ventilation de secours lorsqu'il est détecté que l'électroaimant (5) génère un courant d'induction attractif.

6. Système selon la revendication 1, dans lequel
la vanne de régulation d'écoulement (2) régule un écoulement d'un gaz délivré à un patient selon une opération de régulation reçue, lorsque le mode de ventilation de secours est activé, et stocke un écoulement de base en son sein, lorsque le mode de ventilation de secours est désactivé, de préférence le gaz est de l'oxygène, ou de l'oxygène et de l'air, et de préférence le système comprend en outre un commutateur de système (8), où le commutateur de système (8) est relié à la vanne de régulation d'écoulement (2), et le commutateur de système (8) empêche toute fuite de l'écoulement de base stocké dans la vanne de régulation d'écoulement (2), lorsque le commutateur de système (8) est fermé.

7. Système selon la revendication 1, où :
le système comprend en outre un capteur d'écoulement (9), où le capteur d'écoulement (9) est relié à la vanne de régulation d'écoulement (2) et à la première unité de commande (1), de sorte à détecter un écoulement à travers la vanne de régulation d'écoulement (2) et faire un retour d'information concernant l'écoulement détecté à la première unité de commande (1) ; ou
le système comprend en outre un débitmètre à tube en verre (10), où le débitmètre à tube en verre (10) est relié à la vanne de régulation d'écoulement (2), de sorte à afficher un débit de gaz régulé par la vanne de régulation d'écoulement (2), lorsque le mode de ventilation de secours est activé, de préférence le système comprend en outre un rétroéclairage de tube en verre (11), où le rétroéclairage de tube en verre (11) est disposé au dos du débitmètre à tube en verre (10) et est relié à la première unité de commande (1), et la première unité de commande (1) ordonne au rétroéclairage de tube en verre (11) d'émettre de la lumière lorsqu'elle ordonne d'activer le mode de ventilation de secours, et de préférence, une source d'alimentation pour le rétroéclairage de tube en verre (11) est différente d'une source d'alimentation du dispositif de commande d'écoulement électronique.

8. Système selon la revendication 1, dans lequel :
la première unité de commande (1) est également incluse dans le système de commande d'écoulement électronique, et la première unité de commande (1) détecte un état de fonctionnement du système de commande d'écoulement électronique ; ou
le système de commande d'écoulement électronique comprend une seconde unité de commande (12), où la première unité de commande (1) est reliée à la seconde unité de commande (12) ; et la seconde unité de commande (12) détecte un état de fonctionnement du système de commande d'écoulement électronique et transmet l'état de fonctionnement détecté à la première unité de commande (1), de préférence la première unité de commande (1) détermine que l'état de fonctionnement du système de commande d'écoulement électronique est l'état de défaillance, lorsqu'une interruption de communication avec la seconde unité de commande (12) est détectée.

9. Système selon la revendication 2, dans lequel,
lorsque la première unité de commande (1) connaît une défaillance, l'électroaimant (5) est excité et séparé de l'aimant permanent (6), en commandant ainsi au couvercle (4) de s'ouvrir.

10. Procédé de secours de commande d'écoulement applicable à une machine d'anesthésie, exécuté par un système de secours de commande d'écoulement comprenant une première unité de commande, une vanne de régulation d'écoulement, une cavité de réception et un couvercle, le système de secours de commande d'écoulement étant relié à un système de commande d'écoulement électronique de la machine d'anesthésie, où le procédé comprend les étapes consistant à :
obtenir (S601) un état de fonctionnement du système de commande d'écoulement électronique via la première unité de commande, l'état de fonctionnement du système de commande d'écoulement électronique comprenant un état de défaillance et un état de bon fonctionnement ; et
ordonner (S602), via la première unité de commande, d'activer un mode de ventilation de secours et d'ouvrir le couvercle qui ferme la cavité de réception, de telle sorte qu'une ouverture de la cavité de réception est ouverte pour exposer la vanne de régulation d'écoulement à l'intérieur de la cavité de réception, lorsque le système de commande d'écoulement électronique se trouve dans l'état de défaillance, ou ordonner, via la première unité de commande, de désactiver le mode de ventilation de secours lorsque le système de commande d'écoulement électronique se trouve dans l'état de bon fonctionnement.

11. Procédé selon la revendication 10, dans lequel
le fait d'ordonner, via la première unité de commande, d'activer le mode de ventilation de secours et d'ouvrir le couvercle qui ferme la cavité de réception, lorsque le système de commande d'écoulement électronique se trouve dans l'état de défaillance, ou d'ordonner, via la première unité de commande, de désactiver le mode de ventilation de secours lorsque le système de commande d'écoulement électronique se trouve dans l'état de bon fonctionnement comprend :
le fait d'ordonner, via la première unité de commande, d'exciter et de désexciter un électroaimant selon l'état de fonctionnement du système de commande d'écoulement électronique ;
où un premier aimant parmi un aimant permanent et l'électroaimant est fixé sur le couvercle, l'autre aimant est fixé sur la cavité de réception, l'aimant permanent correspond à l'électroaimant en termes de position, et la première unité de commande est reliée à l'électroaimant.

12. Procédé selon la revendication 11, dans lequel
le fait d'ordonner, via la première unité de commande, d'exciter et de désexciter l'électroaimant selon l'état de fonctionnement du système de commande d'écoulement électronique comprend :
le fait d'ordonner, via la première unité de commande, d'exciter l'électroaimant lorsque le système de commande d'écoulement électronique se trouve dans l'état de défaillance, de telle sorte qu'un champ magnétique généré par un pôle magnétique à une extrémité de l'électroaimant proche de l'aimant permanent est identique à un champ magnétique généré par un pôle magnétique à une extrémité de l'aimant permanent proche de l'électroaimant, de sorte à séparer l'électroaimant de l'aimant permanent, et d'ordonner, via la première unité de commande, de désexciter l'électroaimant lorsque le système de commande d'écoulement électronique se trouve dans l'état de bon fonctionnement.

13. Procédé selon la revendication 10, où le procédé comprend outre :
le fait d'ordonner, via la première unité de commande, d'activer le mode de ventilation de secours lorsqu'un capteur de position détecte que le couvercle est ouvert, de préférence le fait d'ordonner, via la première unité de commande, d'activer le mode de ventilation de secours lorsqu'il est détecté que l'électroaimant génère un courant d'induction séparatif, et d'ordonner, via la première unité de commande, de désactiver le mode de ventilation de secours lorsqu'il est détecté que l'électroaimant génère un courant d'induction attractif.

14. Procédé selon la revendication 10, dans lequel,
si la première unité de commande est également incluse dans le système de commande d'écoulement électronique, le fait d'obtenir l'état de fonctionnement du système de commande d'écoulement électronique via la première unité de commande comprend :
le fait de détecter l'état de fonctionnement du système de commande d'écoulement électronique via la première unité de commande.

15. Procédé selon la revendication 10, où
le système de commande d'écoulement électronique comprend une seconde unité de commande, la première unité de commande étant reliée à la seconde unité de commande, et le fait d'obtenir l'état de fonctionnement du système de commande d'écoulement électronique via la première unité de commande comprend :
le fait de détecter l'état de fonctionnement du système de commande d'écoulement électronique, via la seconde unité de commande, et le fait de recevoir, via la première unité de commande, l'état de fonctionnement détecté par la seconde unité de commande, de préférence le fait d'obtenir l'état de fonctionnement du système de commande d'écoulement électronique via la première unité de commande comprend en outre :
le fait de déterminer, via la première unité de commande, que l'état de fonctionnement du système de commande d'écoulement électronique est l'état de défaillance, lorsqu'une interruption de communication avec la seconde unité de commande est détectée.
